(19) Europäisches Patentamt
European Patent Office
Office européen des brevets

(11) Veröffentlichungsnummer: **0 166 956**
**A1**

# (12) EUROPÄISCHE PATENTANMELDUNG

(21) Anmeldenummer: 85106470.9

(22) Anmeldetag: 25.05.85

(51) Int. Cl.⁴: **C 07 D 233/60**
C 07 D 249/08, A 01 N 43/50
A 01 N 43/653

(30) Priorität: 30.05.84 DE 3420227

(43) Veröffentlichungstag der Anmeldung:
08.01.86 Patentblatt 86/2

(84) Benannte Vertragsstaaten:
AT BE CH DE FR GB IT LI NL SE

(71) Anmelder: BASF Aktiengesellschaft
Carl-Bosch-Strasse 38
D-6700 Ludwigshafen(DE)

(72) Erfinder: Tuerk, Wolfgang, Dr.
Wittelsbachstrasse 61
D-6700 Ludwigshafen(DE)

(72) Erfinder: Sauter, Hubert, Dr.
Neckarpromenade 20
D-6800 Mannheim 1(DE)

(72) Erfinder: Rentzea, Costin, Dr.
Richard-Kuhn-Strasse 1-3
D-6900 Heidelberg(DE)

(72) Erfinder: Meyer, Norbert, Dr.
Dossenheimer Weg 22
D-6802 Ladenburg(DE)

(72) Erfinder: Ammermann, Eberhard, Dr.
Sachsenstrasse 3
D-6700 Ludwigshafen(DE)

(72) Erfinder: Pommer, Ernst-Heinrich, Dr.
Berliner Platz 7
D-6703 Limburgerhof(DE)

## (54) Vinylazole, Verfahren zu ihrer Herstellung und ihre Verwendung bei der Bekämpfung von Pilzen.

(57) Vinylazole der Formel

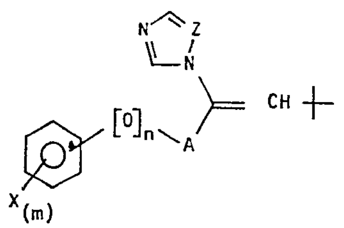

I

in der
A eine Kohlenstoffkette mit 2 bis 10 C-Atomen, die substituiert sein kann und die eine oder mehrere Doppelbindungen, oder eine Dreifachbindung enthalten kann,
X Wasserstoff, Halogen, Trifluormethyl, Alkyl, Alkoxy, gegebenenfalls substituiertes Phenyl oder gegebenenfalls substituiertes Phenoxy,
Z CH oder N
n 0 oder 1
m eine ganze Zahl von 1 bis 5 bedeutet
sowie deren für Pflanzen verträgliche Säureadditionssalze und Metallkomplexe und diese enthaltende Fungizide.

EP 0 166 956 A1

Croydon Printing Company Ltd.

Vinylazole, Verfahren zu ihrer Herstellung und ihre Verwendung bei der Bekämpfung von Pilzen

Die vorliegende Erfindung betrifft neue Vinylazole, Verfahren zu ihrer Herstellung sowie Fungizide, die diese Verbindungen als Wirkstoffe enthalten.

Es ist bekannt, daß Vinylazole, z.B. das 2,2-Dimethyl-4-(1,2,4-triazolyl)-5-phenyl-penten-3, fungizid wirksam sind (vgl. DE-OS 31 03 068). Diese Wirkung befriedigt jedoch nicht.

Es wurde gefunden, daß Vinylazole der Formel I

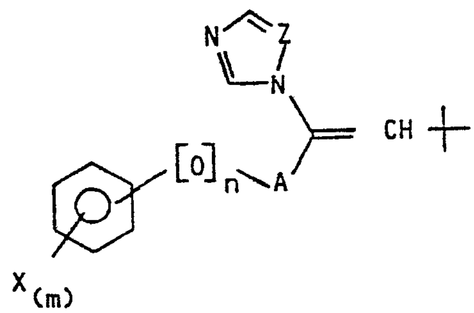

I

in der

A eine Kohlenstoffkette mit 2 bis 10 C-Atomen, die durch 1 bis 3 $C_1$- bis $C_3$-Alkylreste substituiert sein kann, und die eine oder mehrere Doppelbindungen oder eine Dreifachverbindung enthalten kann,

X Wasserstoff, Halogen, Trifluormethyl, $C_1$- bis $C_4$-Alkyl, $C_1$- bis $C_4$-Alkoxy, gegebenenfalls substituiertes Phenyl oder gegebenenfalls substituiertes Phenoxy,

Z CH oder N,

n 0 oder 1,

m eine ganze Zahl von 1 bis 5

bedeutet, wobei X gleich oder verschieden ist, wenn m größer als 1 ist sowie deren für Pflanzen verträgliche Säureadditionssalze und Metallkomplexe eine gute fungizide Wirkung haben, die besser als die Wirkung der bekannten Vinylazole ist.

Die Verbindungen der Formel I können als E/Z-Isomere bezüglich der Doppelbindung vorliegen, die dem Azolrest benachbart ist. Unter der Bezeichnung Vinylazole sind dementsprechend sowohl die reinen Isomeren E und Z als auch ihre Gemische zu verstehen. Sie werden alle von der vorliegenden Erfindung umfaßt.

Sws/P

Die reinen Isomeren bezüglich der C=C-Doppelbindung lassen sich entweder dadurch gewinnen, daß man das bei der Synthese üblicherweise anfallende Isomerengemisch nach bekannten Methoden auftrennt oder in dem man von geeigneten Vorprodukten der Formel II ausgeht, so daß gezielt das E- oder Z-Isomere der Formel I entsteht.

Je nach Beschaffenheit der Kette A können Verbindungen der Formel I zusätzliche Isomere bilden, die sowohl einfach als auch als Gemische vorliegen können und von der vorliegenden Erfindung umfaßt werden.

$C_1$-$C_3$-Alkyl bedeutet beispielsweise Methyl, Ethyl, Propyl.

Der Phenylrest kann beispielsweise durch die folgenden Reste substituiert sein, wobei mehrere Reste identisch oder unterschiedlich sein können.

Wasserstoff, $C_1$-$C_4$-Alkyl, 2-Methyl, 4-Methyl, 3-Methyl, 3-tert.Butyl, 4-tert.Butyl, $C_1$-$C_4$-Alkoxy, 2-Methoxy, 3-Methoxy, 4-Methoxy, 3,5-Dimethoxy, 4-n-Butoxy, 3-Trifluormethyl, 4-Trifluormethyl, 3-Nitro, 4-Nitro, Halogen, 2-Fluor, 3-Fluor, 4-Fluor, 2-Chlor, 3-Chlor, 4-Chlor, 4-Brom, 3-Phenoxy, 4-Phenoxy, 4-Benzyloxy, 2,4-Dichlor, 3,5-Dichlor, 2,6-Dichlor, 2,4,6-Trichlor, $C_1$-$C_4$-Haloalkoxy, 3-Trifluormethoxy, 4-Trifluormethoxy, Halogenphenyl, 3-(4-Chlorphenyl), 4-(3-Chlorphenyl), Halogenphenoxy, 3-(2--Fluorphenoxy), 3-(3-Chlorphenoxy), 3-tert.Butoxy, 4-tert.Butoxy.

A bedeutet beispielsweise $C_2$-$C_8$-Alkylen, Ethylen, Propylen, Butylen, Pentylen, Hexylen, Heptylen, Oktylen, 2-Methylpropylen, 3-Methylpentylen, 2-Methylpentylen, 2-Ethylpropylen, 2-Propylpropylen, 2,4-Dimethylpentylen, 2,3-Buten-1,4-ylen, 2,3-Butin-1,4-ylen.

Man erhält Vinylazole der Formel I beispielsweise durch Abspaltung von HY aus Verbindungen der Formel II

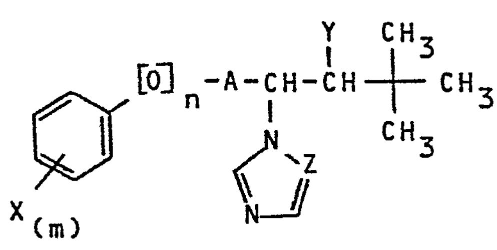

II

in denen A, X, Z, m und n die oben angegebenen Bedeutungen haben und Y eine Abgangsgruppe darstellt. Als Abgangsgruppen für Y kommen beispielsweise Hydroxyl, Tosylat, Mesylat, Halogenid, Acetat, Trifluoracetat und Xanthogenat, in Betracht.

Ist Y eine Hydroxygruppe, so erhält man die Vinylazole der Formel I durch Eliminierung von Wasser in Gegenwart eines sauren Dehydratisierungskatalysators aus den entsprechenden alpha-Azolylalkoholen der Formel II (Y=OH). Geeignete Katalysatoren sind Protonsäuren, Lewis-Säuren, Säureanhydride oder Säurechloride, beispielsweise Schwefel-, Phosphor- oder Ameisensäure, p-Toluolsulfonsäure, Bortrifluorid, Diphosphorpentoxid, Acetanhydrid, Phosphoroxichlorid, Thionylchlorid.

Die Menge an saurem Katalysator beträgt beispielsweise 10 bis 200 Gew.% des alpha-Azolylalkohols II (Y=OH).

Die Wasserabspaltung wird bei einer Temperatur im Bereich zwischen 70 und 180°C, vorzugsweise zwischen 80 und 120°C, durchgeführt. Sie kann in lösungsmittelfreiem Medium durchgeführt werden. Vorzugsweise arbeitet man jedoch in Gegenwart eines Lösungsmittels.

Hierfür geeignete Lösungsmittel sind Toluol, Xylol, Pyridin, Eisessig, Acetonitril.

Der Rest Y kann auch als eine leichter abspaltbare Gruppe, z.B. als Tosylat, Halogenid oder Acetat, vorliegen. Diese Abgangsgruppen lassen sich in Gegenwart eines basischen Katalysators, beispielsweise eines Alkoholats, eines tertiären Amins oder eines Alkalicarbonats, wie Natriummethylat, Natriumethylat, Kalium-tert.butylat, Pyridin, Kaliumcarbonat oder in Gegenwart von Natriumsulfid bei einer Temperatur zwischen 20 und 100°C abspalten.

Man verwendet dabei 1 bis 2,5 Mol an basischem Katalysator pro Mol Verbindung der Formel II.

Die Eliminierungsreaktion wird vorzugsweise in Anwesenheit eines Lösungsmittels durchgeführt. Geeignete Lösungsmittel sind beispielsweise aliphatische Alkohole, wie Ethanol, n-Butanol, tert.-Butanol, Octanol, chlorierte Kohlenwasserstoffe, wie Chloroform, Dichlormethan, N,N-Dialkylsäureamide, wie Dimethylformamid, sowie Dimethylsulfoxid, Pyridin, Toluol, Tetrahydrofuran, Wasser. Auch Gemische dieser Lösungsmittel können verwendet werden.

Ebenso kann der Rest Y auch als Ester, z.B. als eine Acetat- oder Xanthogenatgruppe vorliegen, die thermisch nach bekannten Methoden eliminiert werden kann (Houben-Weyl, Methoden der organischen Chemie, Bd. 5/1b, S. 109ff, Georg Thieme-Verlag, Stuttgart, 1972).

Die Vinylazole der Formel I können in üblicher Weise in für Pflanzen verträgliche Salze oder Metallkomplexe überführt werden. Zur Salzbildung eignen sich Mineralsäuren, wie Salzsäure, Bromwasserstoffsäure, Schwefelsäure, Salpetersäure, Phosphorsäure und organische Säuren, wie Essigsäure und Dodecylbenzolsulfonsäure. Die fungizide Wirksamkeit der Salze der Vinylazole beruht auf dem Kation, so daß das Anion beliebig aus der Vielzahl der pflanzenphysiologisch verträglichen Anionen ausgewählt werden kann.

Metallkomplexe können durch Anlagerung der Vinylazole an die Kationen von Metallsalzen gebildet werden. Hierfür eignen sich insbesondere Kupfer-, Zink-, Eisen-, Mangan- und Nickelsalze, z.B. Kupfer(II)-chlorid, Kupfer(II)-sulfat, Kupfer(II)-nitrat, Zink(II)-chlorid, Eisen(III)-chlorid, Mangan(II)-chlorid, Nickel(II)-bromid.

Die als Ausgangsstoffe verwendeten Verbindungen der Formel II, wobei Y Hydroxy bedeutet, lassen sich nach bekannten Verfahren aus α-Azolylketonen herstellen (DE-OS 30 13 049).

Beispiel 1

a)

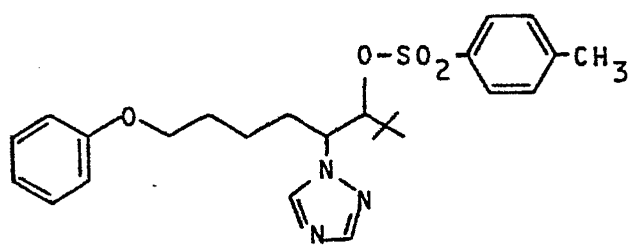

27,1 g 8-(Phenoxy)-2,2-dimethyl-4-(1,2,4-triazol-1-yl)-octanol-3 (diastereomer-einheitlich RR) werden in 250 ml absolutem Dimethylformamid gelöst und unter Rühren bei Raumtemperatur mit 3,4 g 80 %iger Natriumhydriddispersion versetzt. Nach zweistündigem Rühren bei 40 bis 50°C wird auf Raumtemperatur abgekühlt und eine Lösung von 16,2 g Toluol-4-sulfonsäurechlorid in 60 ml Tetrahydrofuran zugetropft; man rührt weitere 12 Stunden bei Raumtemperatur, hydrolysiert mit Wasser, extrahiert mehrfach mit 100 ml Methylenchlorid, trocknet die organische Phase anschließend über Natriumsulfat und engt ein. Durch Kristallisation mit Diethylether erhält man das diastereomer einheitliche Tosylat.

Ausbeute: 12 g (52,5 %)
Schmelzpunkt: 111 bis 112°C

b) 8-Phenoxy-2,2-dimethyl-4-(1,2,4-triazol-1-yl)-octen (3)

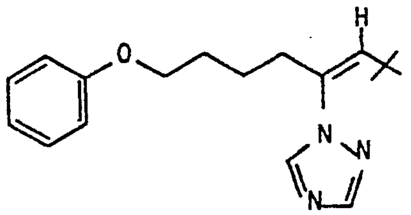

"Z-Isomer"
(Verbindung Nr. 1)

Zu 34,5 g diastereomereinheitlichem Tosylat, gelöst in 370 ml Dimethylsulfoxid, gibt man 44 g gemahlenes Natriumsulfid. Das Reaktionsgemisch zeigt anfangs eine exotherme Reaktion und wird 12 Stunden bei Raumtemperatur nachgerührt. Danach gibt man 500 ml Wasser zu, extrahiert zweimal mit 300 ml Ether, trocknet die organische Phase mit Natriumsulfat und engt ein.

Ausbeute: 8,5 g
Festpunkt: 33 bis 35°C

$^1$H-NMR ($CDCl_3$, 270 MHZ-Spektrum; ppm)
8,05 (s, 2H) Triazol; 7,2 u. 6,9 (m, 5H) Aromat; 5,65 (s, 1H); 3,95 (m 2H); 2,43 (m, 2H); 1,8 u. 1,5 (m, 4H); 0,9 (s, 9H)

Beispiel 2

a)

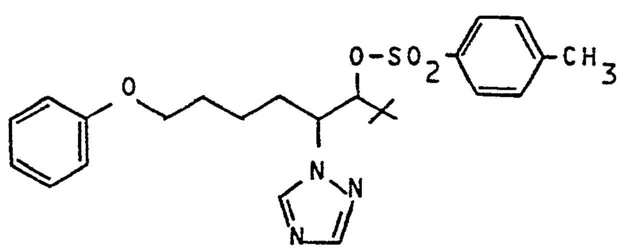

27,1 g 8-(Phenoxy)-2,2-dimethyl-4-(1,2,4-triazol-1-yl)-octanol-3 (diastereomereinheitlich RS) werden in 250 ml absolutem Dimethylformamid gelöst und unter Rühren bei Raumtemperatur mit 3,4 g 80 %iger Natriumhydriddispersion versetzt. Nach zweistündigem Rühren bei 40 bis 50°C wird auf Raumtemperatur abgekühlt und eine Lösung von 16,2 g Toluol-4-sulfonsäurechlorid in 60 ml Tetrahydrofuran zugetropft; man rührt weitere 12 Stunden bei Raumtemperatur, hydrolysiert mit Wasser, extrahiert mehrfach mit 100 ml Methylenchlorid, trocknet die organische Phase anschließend über Natriumsulfat und engt ein. Durch Kristallisation mit Diethylether erhält man das diastereomer einheitliche Tosylat.

Ausbeute: 15,7 g
Schmelzpunkt: 86 bis 88°C

b) 8-Phenoxy-2,2-dimethyl-4-(1,2,4-triazol-1-yl)-octen (3)

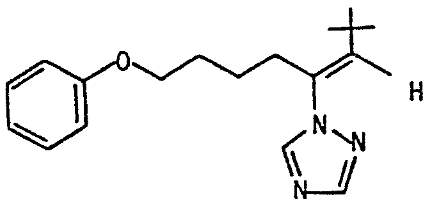

"E-Isomer"
(Verbindung Nr. 2)

Zu 34,5 g diastereomereinheitlichem Tosylat gelöst in 370 ml Dimethylsulfoxid gibt man 44 g gemahlenes Natriumsulfid. Das Reaktionsgemisch zeigt anfangs eine exotherme Reaktion und wird 12 Stunden bei Raumtemperatur nachgerührt. Danach gibt man 500 ml Wasser zu, extrahiert zweimal mit 300 ml Ether, trocknet die organische Phase mit Natriumsulfat und engt im Vakuum ein und isoliert so 7 g der gesuchten Verbindung als Öl.

$^1$H-NMR ($CDCl_3$, 200 MHz; ppm)
8,15 u. 7.92 (s, 2H) Triazol; 7,25 u. 6,85 (m, 5H) Aromat; 5,78 (s, 1H); 3,9 (m, 2H); 2,8 (m, 2H); 1,28 (s, 9H)

Nach dieser Methode können beispielsweise folgende Wirkstoffe hergestellt werden:

0166956

Wirkstoffliste

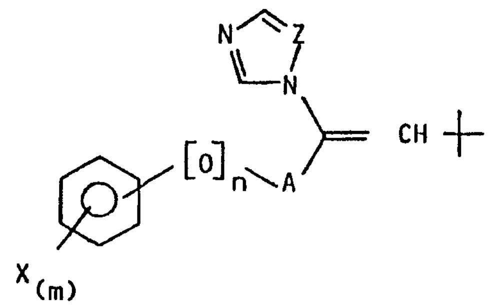

| Nr. | $X_{(m)}$ | $-[O]_n-A-$ | Z | Isomeres Z/E | IR-Daten |
|---|---|---|---|---|---|
| 3 | H | $O-CH_2-CH_2-$ | N | | 2960, 2930, 1601, 1587, 1499, 1474, 1275, 1244, 755, 675 |
| 4 | H | $O-CH_2-CH_2-CH_2-CH_2-$ | H | | 2952, 2869, 1601, 1586, 1498, 1475, 1245, 1079, 755, 692 |
| 5 | H | $O-CH_2-CH_2-CH_2-CH_2-$ | N | Z/E | 2955, 1601, 1499, 1475, 1275, 1245, 1142, 756, 692, 675 |
| 6 | 4-Cl | $O-CH_2-CH_2-CH_2-CH_2-$ | N | | 2956, 2870, 1493, 1474, 1276, 1245, 1171, 1093, 1006, 825 |
| 7 | 2-F | $O-CH_2-CH_2-CH_2-CH_2-$ | N | Z | |
| 8 | 2-F | $O-CH_2-CH_2-CH_2-CH_2-$ | N | E | |
| 9 | H | $O-CH_2-CH=CH-CH_2-$ | N | Z | |
| 10 | H | $O-CH_2-CH=CH-CH_2-$ | N | E | |
| 11 | H | $CH_2-CH_2-CH_2-CH(CH_3)-CH_2-$ | N | | |
| 12 | H | $-(CH_2)_6-$ | N | | |
| 13 | H | $-(CH_2)_7-$ | N | | |
| 14 | H | $-(CH_2)_3-$ | N | | |

| Nr. | $X_{(m)}$ | $-[O]_n-A-$ | Z | Isomeres Z/E | IR-Daten |
|---|---|---|---|---|---|
| 15 | $4-CH_3$ | $-CH_2-CH-CH_2-$<br>$CH_3$ | N | | |
| 16 | 4-tert.butyl | $-CH_2-CH-CH_2-$<br>$CH_3$ | N | | |
| 17 | $2,4-Cl_2$ | $-CH_2-CH-CH_2-$<br>$CH_3$ | N | | |
| 18 | 4 F | $-CH_2-CH-CH_2-$<br>$CH_3$ | N | | |
| 19 | $4-OCH_3$ | $-CH_2-CH-CH_2-$<br>$CH_3$ | N | | |
| 20 | H | $-(CH_2)_4-$ | N | | |
| 21 | $4-CH_3$ | $-(CH_2)_5-$ | N | | |
| 22 | 4-Cl | $-(CH_2)_5-$ | N | | |
| 23 | H | $-(CH_2)_2-CH-(CH_2)_2-$<br>$CH_3$ | N | | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Cercospora musae an Bananen,
Pseudocercosporella herpotrichoides an Weizen, Gerste,
Pyricularia oryzae an Reis,
Hemileia vastatrix an Kaffee,
Alternaria solani an Kartoffeln, Tomaten,
Verticillum-Arten in Baumwolle und Gemüse.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit

0136956

Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle, (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz u.a. zur Bekämpfung holzzerstörender Pilze wie Coniophora puteanea und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung Nr. 1 mit 10 Gewichtsteilen N-Methyl-alpha-pyrrolidon und erhält eine Lösung, die zur Anwendung in Form kleinster Tropfen geeignet ist.

II. 20 Gewichtsteile der Verbindung Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von

40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung Nr. 5 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gewichtsteile der Verbindung Nr. 6 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gewichtsteile der Verbindung Nr. 1 werden den mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI. 3 Gewichtsteile der Verbindung Nr. 2 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung Nr. 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung Nr. 5 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung Nr. 6 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den neuen Verbindungen kombiniert werden können, sind beispielsweise:

Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,

heterocyclische Strukturen, wie
2-Heptadecyl-2-imidazolin-acetat
2,4-Dichlor-6-(o-chloranilino)-s-triazin
O,O-Diethyl-phthalimidophosphonothioat
5-Amino-1-(bis-(dimethylamino)-phosphinyl)-3-phenyl-1,2,4-triazol

2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
2-Methoxycarbonylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethylthio-phthalimid

N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Iod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-
-triazol
1[2-(2,4-Dichlorphenyl)-4-n-propyl-1,3-dioxolan-2-yl-ethyl]-1-H-1,2,4-
-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
alpha-(2-Chlorphenyl)-alpha-(4-chlorphenyl)-5-pyrimidin-methanol
5-Butyl-2-dimethylamino-4-hyroxy-6-methyl-pyrimidin

Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

sowie verschiedene Fungizide, wie
Dodecylguanidinacetat
3-(3-(3,5-Dimethyl-2-oxycyclohexyl)-2-hydroxyethyl)-gluataramid
Hexachlorbenzol
DL-Methyl-N-(2,6-dimethyl-phenyl)-N-furoyl(2)-alaninat,
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl(-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid
2-Cyano-N-(ethylaminocarbonyl)-2-methoximino-acetamid
1-(2-(2,4-Dichlorphenyl)-pentyl)-1H-1,2,4-triazol
2,4-Difluor-alpha-(1H-1,2,4-triazolyl-1-methyl)-benzhydrylalkohol

Für die folgenden Versuche wurde als Vergleichswirkstoff die bekannte Verbindung 2,2-Dimethyl-4-(1,2,4-triazolyl)-5-phenyl-penten-3 (A) verwendet.

Versuch 1

Wirksamkeit gegen Gurkenmehltau

Blätter von in Töpfen gewachsenen Gurkenkeimlingen der Sorte "Chinesische Schlange" werden im Zweiblattstadium mit einer Sporensuspension des Gurkenmehltaus (Erysiphe cichoracearum) besprüht. Nach etwa 20 Stunden werden die Versuchspflanzen mit wäßriger Spritzbrühe, die 80 % Wirkstoff und 20 % Emulgiermittel in der Trockensubstanz enthält, bis zur Tropfnässe besprüht. Nach dem Antrocknen des Spritzbelages werden sie anschließend im Gewächshaus bei Temperaturen zwischen 20 und 22°C und 70 bis 80 % relativer Luftfeuchtigkeit aufgestellt. Zur Beurteilung der Wirksamkeit der neuen Stoffe wird das Ausmaß der Pilzentwicklung nach 21 Tagen beurteilt.

Das Ergebnis des Versuches zeigt, daß die Verbindungen 1, 2, 5 und 6 bei der Anwendung als 0,025 %ige Wirkstoffbrühe eine bessere fungizide Wirkung (z.B. 100 %) zeigen als der Wirkstoff A (z.B. 70 %).

Versuch 2

Wirksamkeit gegen Botrytis cinerea an Paprika

Paprikasämlinge der Sorte "Neusiedler Ideal Elite" werden, nachdem sich 4 bis 5 Blätter gut entwickelt haben, mit wäßrigen Suspensionen, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß der Wirkstoff 5 bei der Anwendung als 0,05 %ige Wirkstoffbrühe eine bessere fungizide Wirkung (z.B. 97 %) zeigt als der Wirkstoff A (z.B. 70 %).

Patentansprüche

1. Vinylazol der Formel

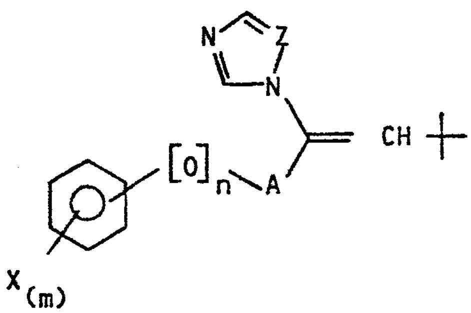

I

in der

A eine Kohlenstoffkette mit 2 bis 10 C-Atomen, die durch 1 bis 3 $C_1$- bis $C_3$-Alkylreste substituiert sein kann und die eine oder mehrere Doppelbindungen oder eine Dreifachbindung enthalten kann,

X Wasserstoff, Halogen, Trifluormethyl, $C_1$- bis $C_4$-Alkyl, $C_1$- bis $C_4$-Alkoxy, gegebenenfalls substituiertes Phenyl oder gegebenenfalls substituiertes Phenoxy,

Z CH oder N

n 0 oder 1

m eine ganze Zahl von 1 bis 5 bedeutet, wobei X gleich oder verschieden ist, wenn m größer als 1 ist,

sowie deren für Pflanzen verträgliche Säureadditionssalze und Metallkomplexe.

2. Verfahren zur Herstellung von Vinylazolen der Formel I gemäß Anspruch 1, dadurch gekennzeichnet, daß man aus Verbindungen der Formel II

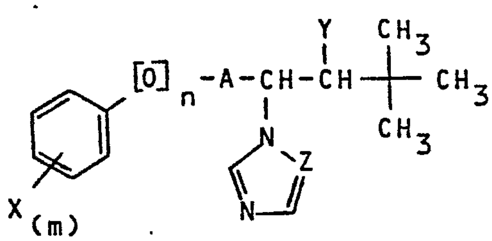

II

in der A, Z, X, m und n die in Anspruch 1 angegebenen Bedeutungen haben und Y eine Abgangsgruppe darstellt, HY eliminiert und die so erhaltenen Verbindungen gegebenenfalls in ihre Säureadditionssalze oder Metallkomplexe überführt.

3. Fungizide Mittel, enthaltend eine Verbindung gemäß Anspruch 1.

4. Fungizide Mittel, enthaltend eine Verbindung gemäß Anspruch 1 und einen festen oder flüssigen Trägerstoff.

5. Verfahren zur Herstellung von Fungiziden, dadurch gekennzeichnet, daß man eine Verbindung der Formel I gemäß Anspruch 1 mit festen oder flüssigen Trägerstoffen mischt.

6. Verfahren zur Bekämpfung von Fungi, dadurch gekennzeichnet, daß man eine Verbindung der Formel I gemäß Anspruch 1 auf diese einwirken läßt.

7. Verfahren zur vorbeugenden Bekämpfung von Fungi, dadurch gekennzeichnet, daß man eine Verbindung der Formel I gemäß Anspruch 1 auf durch Pilzbefall bedrohte Flächen, Pflanzen oder Saatgüter einwirken läßt.

8. Vinylazol gemäß Anspruch 1, dadurch gekennzeichnet, daß A den $C_4H_8$--Rest, X Wasserstoff oder Fluor, Z Stickstoff, n die Zahl 1 und m die Zahl 1 bedeuten.

Patentansprüche

1. Fungizid, enthaltend ein Vinylazol der Formel

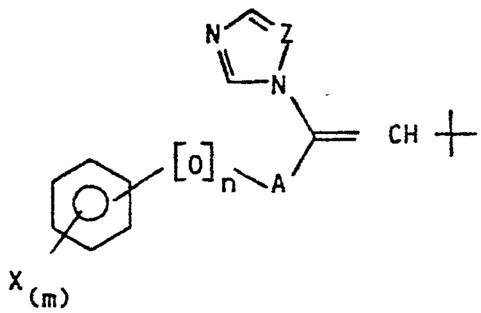

I

in der

A eine Kohlenstoffkette mit 2 bis 10 C-Atomen, die durch 1 bis 3 $C_1$- bis $C_3$-Alkylreste substituiert sein kann und die eine oder mehrere Doppelbindungen oder eine Dreifachbindung enthalten kann,

X Wasserstoff, Halogen, Trifluormethyl, $C_1$- bis $C_4$-Alkyl, $C_1$- bis $C_4$-Alkoxy, gegebenenfalls substituiertes Phenyl oder gegebenenfalls substituiertes Phenoxy,

Z CH oder N

n 0 oder 1

m eine ganze Zahl von 1 bis 5 bedeutet, wobei X gleich oder verschieden ist, wenn m größer als 1 ist,

sowie deren für Pflanzen verträgliche Säureadditionssalze und Metallkomplexe.

2. Verfahren zur Herstellung von Vinylazolen der Formel I gemäß Anspruch 1, dadurch gekennzeichnet, daß man aus Verbindungen der Formel II

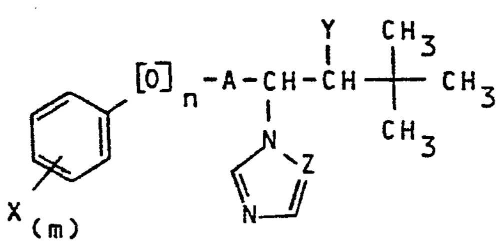

II

in der A, Z, X, m und n die in Anspruch 1 angegebenen Bedeutungen haben und Y eine Abgangsgruppe darstellt, HY eliminiert und die so erhaltenen Verbindungen gegebenenfalls in ihre Säureadditionssalze oder Metallkomplexe überführt.

3. Fungizide Mittel, enthaltend eine Verbindung gemäß Anspruch 1 und einen festen oder flüssigen Trägerstoff.

4. Verfahren zur Herstellung von Fungiziden, dadurch gekennzeichnet, daß man eine Verbindung der Formel I gemäß Anspruch 1 mit festen oder flüssigen Trägerstoffen mischt.

5. Verfahren zur Bekämpfung von Fungi, dadurch gekennzeichnet, daß man eine Verbindung der Formel I gemäß Anspruch 1 auf diese einwirken läßt.

6. Verfahren zur vorbeugenden Bekämpfung von Fungi, dadurch gekennzeichnet, daß man eine Verbindung der Formel I gemäß Anspruch 1 auf durch Pilzbefall bedrohte Flächen, Pflanzen oder Saatgüter einwirken läßt.

Europäisches Patentamt

# EUROPÄISCHER RECHERCHENBERICHT

0166956
Nummer der Anmeldung

EP 85 10 6470

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| Y | EP-A-0 057 365 (BASF AG) * Seite 1, Zeile 12 - Seite 4, Zeile 34, besonders Seite 3, Zeilen 5-7; Seite 8, Zeile 1 - Seite 10, Zeile 19 * & DE - A - 3 103 068 (Kat. D) | 1-6 | C 07 D 233/60<br>C 07 D 249/08<br>A 01 N 43/50<br>A 01 N 43/653 |
| Y | EP-A-0 040 350 (BASF AG) * Seite 8, Zeilen 1-25; Seite 10, Zeile 13 - Seite 11, Zeile 3 * | 1-6 | |
| A | EP-A-0 015 387 (BAYER AG) * Seite 2, Zeile 18 - Seite 3, Zeile 22; Seite 10, Zeile 1 - Seite 13, Zeile 20; Seite 49, Zeile 17 - Seite 51, Zeile 31 * | 1,3-6 | |
| A | EP-A-0 076 445 (BASF AG) * Ansprüche * | 1,3-6 | RECHERCHIERTE SACHGEBIETE (Int. Cl.4) |
| | | | C 07 D 233/00<br>C 07 D 249/00<br>C 07 D 521/00<br>A 01 N 43/00 |

Der vorliegende Recherchenbericht wurde für alle Patentansprüche erstellt.

| Recherchenort | Abschlußdatum der Recherche | Prüfer |
|---|---|---|
| BERLIN | 07-08-1985 | VAN AMSTERDAM L.J.P. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument